# EUROPEAN PATENT APPLICATION

(11) **EP 4 311 549 A1**
(43) Date of publication of application: **31.01.2024**
(21) Application number: 22187487.8
(22) Date of filing: 28.07.2022
(51) Int. Cl.: A61K 31/198, A61P 43/00

(54) **COMPOSITION COMPRISING CREATINE FOR USE IN THE TREATMENT OF POST VIRAL FATIGUE SYNDROME**

(71) Applicant: AlzChem Trostberg GmbH, 83308 Trostberg (DE)
(72) Inventor: Bezler, Jürgen, 84489 Burghausen (DE); Löber, Verena, 83376 Truchtlaching (DE); Ostojic, Sergej M., 26000 Pancevo (RS); ALBER, Robert, 83209 Prien (DE)

(57) **Abstract**

The present invention relates to a pharmaceutical composition comprising creatine or physiologically acceptable derivatives and/or salts and or adducts thereof for use in the treatment of PVFS, in particular associated with mental fatigue, reduced motivation, reduced activity, or concentration difficulties. A further embodiment of the invention relates to the use of a composition comprising creatine or physiologically acceptable derivatives and/or salts and or adducts thereof as dietary supplement for preparation of a diet supporting the recovery from PVFS.

## Description

### Field of the invention:

The present invention is related to pharmaceutical compositions comprising creatine for use in the treatment of post viral fatigue syndrome caused by virus infection of the lungs or the lower respiratory system in a subject in need thereof. A further object of the invention is the use of creatine or creatine derivatives as dietary supplement for the preparation of a diet for patients suffering under PVFS.

### Description of the prior art:

Although COVID-19 is seen as a disease that primarily affects the lungs, it can also damage other organs, including the heart, the vascular system, the kidneys and the brain for example. Organ damage increases the risk of sequelae, such as cognitive impairment, heart complications (myocarditis), chronic kidney impairment, stroke, thrombosis, and Guillain-Barre syndrome.

However, most people who are infected with SARS-CoV-2 recover within a few weeks. But some people, even those who had mild versions of the disease, continue to experience a brought variety of symptoms after their initial recovery.

Typical symptoms that may persist after a SARS-CoV-2 infection are for example fatigue (e.g. post-viral fatigue syndrome (PVFS) or chronic fatigue syndrome (CFS)), breathing difficulties or breathlessness (dyspnea), lung or chest pain, joint pain, muscle pain or headache, concentration and memory difficulties, sleep problems (insomnia), loss of smell (anosmia) or taste (ageusia) etc., wherein fatigue, breathing difficulties (dyspnea) and chest pain belongs to the most reported conditions after acute SARS-CoV-2 infection.

PVFS is a complex long-term disorder, characterized by an inability to participate in routine activities in daily life that were possible before the virus infection, which lasts for at least 3 months. Besides other negative consequences, coronavirus infections are often linked with PVFS.

In serious courses of disease, about 70% of COVID-patients suffer from fatigue in the acute phase of the infection, wherein the symptom often persists after recovery of the patients from infection (Manal S., Barnett J., Brill S. et al. (Thorax, 2021; 76, 396-398), Halpin S.J., Mclvor C., et al. (J. Med. Virol. 2021; 93, 1013-1022, Carfi A., et al. JAMA, Vol. 324, No. 6, 603-605, 2020).

The described conditions are summarized under the term "Post-COVID-19 syndrome" or "Long-COVID-19", hereinafter referred to as Post-COVID.

Persons with post viral fatigue syndrome are complaining of physical and mental exhaustion, including reduced activity, reduced motivation, poor memory, concentration difficulties, unrefreshing sleep, emotional lability and in some cases depression. Recommendations for these persons are usually limited to behavioral advices, such as keeping exercise low at the beginning and increasing activity slowly, maintaining a routine of daily activities (eating, sleeping etc.) to support recovery.

Practical medical treatment strategies of patients suffering from Long-COVID are summarized in Deutsches Ärzteblatt 2020; 49, 117 and are based on primary care recommendations of Long-Covid as described by Greenhalgh, T.; Knight M., A'Court C.; BMJ 2020; 370, 3026.

Moscatelli, F. et al., (Nutrients 2021; 13, 976-988) discuss the role of nutritional inventions and highlight the fact that strong data from clinical trials are needed to support any such assumption. Adequate nutrition is required to support the immune cell function by allowing to engage robust responses to pathogens. The micronutrients with the strongest evidence for immune support are vitamins C, D and zinc.

Creatine is a methylguanidinoacetic acid usually available from animal-based foods and/or produced naturally in the body from the amino acids arginine, glycine and methionine. Endogenous creatine synthesis provides about half of the daily need. The remaining amount of creatine needed to maintain normal tissue levels of creatine is obtained in the diet from animal based products such as fish or meat or from dietary supplements. Creatine plays a vital role in the energy metabolism in every cell of the body. It mainly serves as a metabolic intermediary of energy transfer by facilitating the recycling of ATP, the source of energy for use and storage at the cellular level. Thus, creatine is found in high concentrations in organs with high energy turnover, with ~95% of the human body's creatine stores in the skeletal muscle and the remaining 5% in the brain, liver, kidney, and testes (McCall, W., Persky, AM. Pharmacokinetics of creatine. Subcell Biochem 2007, 46, 261-273; Bonilla, D.A. et al., Metabolic Basis of Creatine in Health and Disease: A Bioinformatics-Assisted Review. Nutrients 2021, 13, 1238; Brosnan, M.E. et al., The role of dietary creatine. Amino Acids 2016, 48, 1785-1791; Harris, R. Creatine in health, medicine and sport: An introduction to a meeting held at Downing College, University of Cambridge, July 2010, Amino Acids 2011, 40, 1267; Harris, R.C.et al. Elevation of creatine in resting and exercised muscle of normal subjects by creatine supplementation. Clin. Sci. 1992, 83, 367-374; Kreider, R.B.; Stout, J.R. Creatine in Health and Disease. Nutrients 2021, 13, 447; Ostojic, S.M.; Forbes, S.C. Perspective: Creatine, a Conditionally Essential Nutrient: Building the Case. Adv. Nutr. 2021, 00, 1-4).

The administration of creatine has therefore been considered as a supportive measure for the treatment of a variety of diseases. In particular Ostojic, S.M. et al. for example describes a dietary treatment of chronical fatigue syndrome (CFS) comprising guanidinoacetic acid (GAA) (Nutrients 2016, 8, 72). The potency of creatine in the treatment of post-viral fatigue syndrome (PVFS) is discussed in Ostojic, S.M. Nutrients 2021, 13, 503; Kreider R.B. et al., Nutrients 2021, 13, 447.

Starting from this the problem to be solved by the present invention is to improve the recovery from post viral fatigue syndrome (PVFS) caused e.g. by SARS-CoV-2 virus, particularly of patients suffering under PVFS associated with mental fatigue, reduced motivation, reduced activity, or concentration difficulties.

### Description of the invention:

The problem is solved by administration of creatine in patients in need thereof. Creatine supports the recovery from PVFS, particularly when PVFS is caused by SARS-CoV-2 virus. Especially the mental status of patients suffering under PVFS can be improved by administration of creatine, whereby the time to exhaustion can be increased. The mental status of patients comprises parameters such as mental fatigue, reduced motivation, and concentration difficulties.

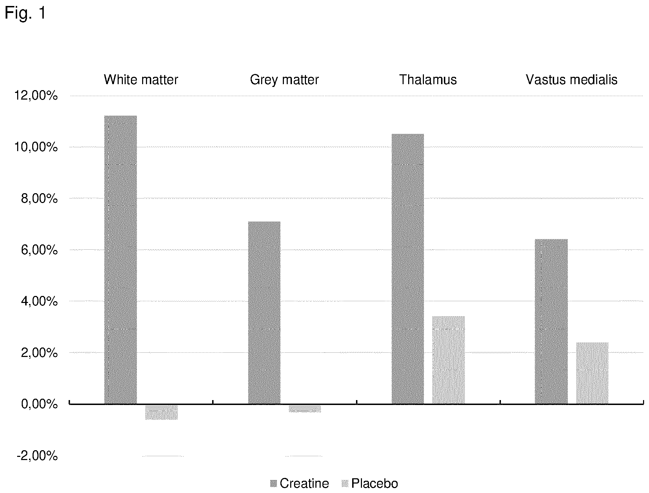

The effect is supported by a strong enrichment of creatine in the thalamus, grey matter and particularly white matter of the brain in patients suffering under Post-COVID (see Fig. 1). The enrichment is achieved by the supplemental administration of creatine in patients in need thereof.

Therefore, a first embodiment of the invention is a pharmaceutical composition comprising creatine or physiologically acceptable derivatives and/or salts and or adducts thereof for use in the treatment of PVFS according to claim 1, in particular of PVFS caused by SARS-CoV-2.

A second embodiment of the invention is the use of creatine or physiologically acceptable derivatives and/or salts and or adducts thereof as dietary supplement or as a supplement for preparation of a diet supporting the recovery from PVFS, in particular when the PVFS is caused by SARS-CoV-2 and when the PVFS is associated with symptoms selected from the group of mental fatigue, reduced motivation, or concentration difficulties.

A further embodiment of the present invention is the administration of creatine in combination with glucose. Glucose supports the uptake of creatine in the brain compared to the ingestion of creatine alone.

In a further embodiment of the invention the pharmaceutical composition or the dietary supplement comprising creatine or physiologically acceptable derivative and/or salt and or adduct thereof is used for the treatment of PVFS in combination with breathing exercises. Pulmonary rehabilitation is performed in accordance with Wang T.J. et al., Am J Phys Med Rehabil, 2020, Jun 11 (DOI 10.1097/ PHM. 0000000000001505 / PMCID: 7315835). Pulmonary rehabilitation is tailored to each individual patient and includes for example modified segmental breathing, breathing exercises for strengthening pulmonary muscles, bed mobility exercise, stretching, gymnastics, and/or walking. The extent of the exercises is low at the beginning and is slowly increased without exposing the patient higher efforts. The exercise frequency is e.g. 2 to 4 times per day for 10 to 15 minutes. The exercise time can be increased slowly. Pulmonary rehabilitation measures, such as training of the respiratory musculature and the muscles of exhalation by breathing exercises should be started as soon as possible, provided that the patient's state of health permits this. The rehabilitation is usually started within 20 weeks, preferably within 12 weeks, most preferably within 6 weeks, after infection has subsided.

The pharmaceutical composition of the first embodiment and the composition used as dietary supplement of the second embodiment are referred to herein after also as creatine composition, creatine comprising composition or composition comprising creatine. The term "creatine composition", creatine comprising composition" or "composition comprising creatine" comprise also physiologically acceptable creatine derivatives, creatine salts and/or creatine adducts if not expressly stated otherwise.

The creatine comprising compositions of the present invention are particularly useful to support recovery from fatigue syndrome after virus infection such as SARS-CoV-2 infection, particularly when the post viral fatigue syndrome is associated with mental fatigue, reduced motivation, and concentration difficulties. However, PVFS is one of the most occurring condition of Post-COVID. A fatigue syndrome often persists for weeks, months or even longer in patients with an overcome SARS-CoV-2 infection and can compromise general health significantly.

Several acute and chronic viral infections which cause PVFS, include coronaviruses (e.g. SARS, MERS, SARS-CoV-2), Epstein-Barr virus, cytomegalovirus, and coxsackieviruses. A post viral fatigue syndrome can persist for weeks or months. After a SARS-CoV-2 infection, for example, PVFS can persist up to a year or even longer.

The recovery of patients suffering under PVFS can be surprisingly improved by administration of creatine, in particular in combination with glucose. Creatine is also known as methylguanidinoacetic acid, which is naturally occurring in the body of animals and humans. Other names for creatine are N-(Aminoiminiomethyl)-N-methylglycine or N-Methyl-N-guanylglycine. Creatine is further available from animal-based foods or in higher amounts as food supplement. In food supplements preferably creatine monohydrate is used, which can be prepared in very high purity.

Beside creatine, also physiologically acceptable creatine derivatives can be used in accordance with the invention. Such creatine derivatives can be natural occurring compounds, such as creatine phosphate, or pro-drugs of creatine, which are able to release creatine under physiological conditions, such as creatine esters. Physiologically acceptable creatine derivatives are preferably selected from the group consisting of creatine, creatine hydrates, creatine esters or amides, including creatine C₁-C₅-alkyl esters, N-C₁-C₅-alkyl amides, creatine phosphate, creatinol-O-phosphate or a mixture thereof.

Suitable creatine salts, creatine adducts, salts of physiologically acceptable creatine derivatives and adducts of the physiologically acceptable creatine derivatives are preferably selected from the group consisting of the corresponding acetates, citrates, maleates, fumarates, tartrates, malates, pyruvates, ascorbates, succinates, aspartates, lactates, oxalates, formates, benzoates, phosphates, sulfates, chlorides, hydrochlorides, of the corresponding potassium, sodium, calcium, magnesium salts, of the corresponding L-carnitine, acetyl-L-carnitine, taurine, betaine, choline, methionine adducts or a mixture thereof.

Treatment with creatine can already begin during the virus infection, preferably within about three months (12 weeks) after infection. The duration of supplementation of creatine lasts normally between 1 week and 18 months or longer, preferably between 1 and 12 months, in particular between 3 and 8 months depending on the symptoms of the subject in need thereof.

The amount of creatine to be administered is in the range of 3g to 30g per day. Preferably the dosage is in the range of 7g to 25g, most preferred between 8g and 20g, which is higher than commonly recommended for sportsmen or sportswomen.

Preferably the administration of creatine is divided in an accumulation phase and a maintaining phase, wherein the daily dose of creatine in the composition is in the range of 10g to 30g in the initial accumulation phase and in the range of 7g to 15g for the subsequent maintaining phase. The accumulation phase has a duration of up to 3 weeks, preferably between 3 and 14 days, particularly between 5 and 10 days and the maintaining phase has a duration of 1 week to 18 months, preferably between 2 and 12 months, in particular between 3 and 8 months.

The accumulation phase is usually the initial phase. However, additional accumulation phases, for example with a duration between 1 day and 7 days, may be integrated into the maintaining phase.

The daily creatine dose can be administered once a day, during breakfast for example, or spread over 2, 3, 4 or 5 times a day.

If the administered creatine composition comprises a creatine derivative, pro drug, adduct or salt, the amount of the creatine moiety contained therein is decisive for the daily dosage within the ranges described above.

The creatine composition described herein may be preferably administered orally, in form of tablets, coated tablets, capsules, granules or powders.

In particular, granulates or powders comprising the creatine composition are used as aqueous suspension or in water-soluble form. The solubility of pure creatine and several creatine derivatives is low. Creatine for example has a solubility of 17 g/L (at 20°C). Creatine and derivatives thereof with low solubility can be used in form of an aqueous suspension. A disadvantage of an aqueous suspension is often, that it segregates before ingestion. Therefore water-soluble granules or powders are usually preferred. To increase the water solubility of creatine or creatine derivatives water soluble salts or adducts thereof may be used. To increase the water-solubility particularly the use of acids or complexing agents may be useful to provide the corresponding creatine salts or creatine derivative salts. Examples for suitable acids, including carboxylic acids, and complexing agents are selected from the group malic acid, aspartic acid, ascorbic acid, succinic acid, pyruvic acid, fumaric acid, gluconic acid, alpha-ketoglutaric acid, oxalic acid, acetic acid, formic acid, sulfuric acid, hydrochloric acid, L-carnitine, acetyl-L-carnitine, taurine, betaine, choline, and lipoic acid. Peptides and amino acids may be also useful to increase the solubility of creatine and creatine derivatives. Further sodium, potassium, calcium and magnesium salts may be used to increase the water-solubility of creatine or creatine derivatives.

The molar ratio of creatine or the creatine derivatives and the acids or complexing agents mentioned above is usually in the range of 5:1 to 1:5, preferably in the range of 2:1 to 1:2, in particular in the range of 1.3:1 to 1:1.3.

The granules and powders can also be used for preparing foodstuff supporting the recovery from PVFS, in particular by improving the mental status of patients suffering under PVFS.

The creatine composition used in accordance with the present invention may be applied further in tablet form.

The creatine composition may be tableted as such or in form of a formulation comprising excipients. Suitable excipients are for example pharmacologically inactive ingredients, such as binders, fillers, antioxidants, preservatives, stabilizers, anti-caking agents, lubricants, disintegrants, flavors, pigments etc.

As binder or filler a wide variety of compounds may be used. Dibasic calcium phosphate; saccharides, including lactose and sucrose; polysaccharides and derivatives thereof, including starches, cellulose, modified cellulose, and cellulose ethers, such as hydroxypropyl cellulose or hydroxyethyl cellulose; microcrystalline cellulose; sugar alcohols, such as xylitol, sorbitol or mannitol; peptides, such as gelatin; polymers, e.g. polyvinylpyrrolidone, polyethylene glycol, etc. are common binders or fillers for tablets.

Typical suitable preservatives are for example cysteine, methionine, citric acid, sodium citrate, tatrazines or synthetic preservatives like parabens, including methyl paraben and propyl paraben, and benzoic acid. Suitable antioxidants may be selected from group of vitamin A, vitamin C, vitamin E, retinyl palmitate, and selenium.

Lubricants and anti-caking agents reduce the adhesion in granulates or powders and thus prevent sticking to tablet punches. They are also used to help protect tablets from sticking. The most commonly used anti-caking agents is magnesium stearate, stearic acid, or stearin, but also other magnesium or calcium salts of fatty acids may be used instead or in addition. Common mineral lubricants for example are talc or silica.

Disintegrants expand and dissolve when wet causing a tablet to break apart in the digestive tract, or in specific segments of the digestion process, releasing the ingredients for absorption by the body. Examples of disintegrants include crosslinked polymers like crosslinked polyvinylpyrrolidone (crospovidone) and crosslinked sodium carboxymethyl cellulose (croscarmellose sodium). Other suitable disintegrants are for example modified starch or sodium starch glycolate.

Flavors can be used to mask unpleasant tasting tablet ingredients. Further the ingredients may increase the patients' acceptance of the tablets. Flavorings may be natural, e.g. fruit extracts, or artificial. Natural extracts of vanilla, peach, apricot, raspberry, mint, anise or cherry can be used as flavors, for example. Antacid compounds or cough syrup would be also suitable.

Suitable pigments and colours are for example food colourants.

Tablet coatings protect tablet ingredients from deterioration by moisture in the air and make large or unpleasant-tasting tablets easier to swallow. For most coated tablets, a cellulose ether, particularly hydroxypropyl methylcellulose (HPMC) film coating is used. But other coating materials are also useful, for example synthetic polymers, shellac, plant fibers, waxes, fatty acids or polysaccharides. Capsules are coated usually with gelatine.

Particularly useful coatings for tablets related to the invention disclosed herein are crosslinked sodium carboxymethyl cellulose (croscarmellose sodium), silicified microcrystalline cellulose, agglomerated anhydrous lactose and lactitol monohydrate.

Preferred tablets comprise at least
a) 10 wt.-% to 100 wt.-%, preferably 30 wt.-% to 99 wt.-% creatine, creatine derivatives or salts or adducts thereof;
b) 0 to 80 wt.-% carbohydrates, including preferably glucose;
c) 0 to 20 wt.-% anti-caking agents selected from fatty acids or fatty acid salts, particularly magnesium stearate;
d) 0 to 20 wt.-% acidifier, preferably citric acid;
e) 0 to 20 wt.-% fatty acids, preferably coconut oil;
f) 0 to 5 wt.-% flavors.

Preferred capsules comprise at least
a) 10 wt.-% to 95 wt.-%, preferably 30 wt.-% to 80 wt.-% creatine, creatine derivatives or salts or adducts thereof
b) 0 to 80 wt.-% carbohydrates, including preferably glucose;
c) 0 to 20 wt.-% anti-caking agents selected from fatty acids or fatty acid salts, particularly magnesium stearate,
d) 0 to 5 wt.-% flavors;
e) 5 to 90 wt.-% gelatin.

The described ingredients may be useful not only in tablets but also in granules or powders comprising creatine or creatine derivatives or salts or adducts thereof. Particularly binders, fillers, antioxidants, preservatives, stabilizers, anti-caking agents, lubricants, disintegrants, flavors, pigments, and carbohydrates can be part of the creatine composition used in accordance with the present invention. Preferably the granules or powders comprises a combination of creatine and glucose.

Preferred granules or powders relating to the described invention comprise 10 wt.-% to 100 wt.-%, preferably 30 wt.-% to 99 wt.-% creatine, creatine derivatives or salts or adducts thereof.

Particularly preferred tablets, capsules, granules or powders comprising between 10 to 80 wt.-% glucose, preferably between 30 and 70 wt.-%. The preferred ratio of creatine : glucose in the compositions is in the range of 1 : 3 to 3 : 1, most preferred between 1 : 2 and 2 : 1.

Creatine and glucose can be formulated together e.g. in tablets, capsules, or granules, but the combination can also be administered in form of mixture of the compounds, e.g. in powders. A further possibility is a separate intake of creapure and glucose, preferably with a time lag of below 2 hours, most preferably below 1 hour or even below 30 minutes. Advantageously creatine and glucose are administered together. However, further preferred is the intake of creatine in a first step before glucose is administered in a second step.

In combination with creatine, creatine derivatives or salts or adducts thereof, anti-inflammatory drugs can be applied. Anti-inflammatory drugs are for example non-steroidal drugs, such as aspirin, ibuprofen, naproxen, diclofenac, celecoxib, mefenamic acid, etoricoxib, indomethacin etc. or steroids, such as corticosteroids, including cortisone, hydrocortisone and prednisone.

Further combinations of creatine, creatine derivatives or salts or adducts thereof, with neuroprotective drugs can be advantageous. Preferred neuroprotective agents include glutamate excitotoxicity inhibitors such as ginsenoside, riluzole, progesterone, estrogen, memantine, or simvastatin; stimulants such as caffeine; growth factors such as IGF-1, CNTF; nitric oxide synthase inhibitors; and caspase inhibitors or erythropoietin.

A combination of creatine, creatine derivatives or salts or adducts thereof, with drugs for the treatment of the virus infection is also possible, in particular if the administration of creatine is started during the acute phase of the virus infection.

For the treatment of COVID-19 drugs of the group Lagevrio (molnupiravir), Olumiant 20 (baricitinib), tixagevimab / cilgavimab, Kineret (anakinra), Paxlovid (PF-07321332 / ritonavir), Regkirona (regdanvimab), RoActemra (tocilizumab), Ronapreve (casirivimab / imdevimab), Veklury (remdesivir), Xevudy (sotrovimab) are available for example. For the treatment of influenza Rapivab (peramivir), Relenza (zanamivir), Tamiflu (oseltamivir phosphate), Xofluza (baloxavir marboxil) are suitable drugs. For 25 the treatment of adenoviruses cidofovir, ribavirin, ganciclovir, and vidarabine, are useful drugs.

Creatine, creatine derivatives or salts or adducts thereof are preferably used in combination with a pain-reducing, anti-inflammatory diet according to a preferred embodiment of the present invention. The preferred diet shall be provide all nutrients for normal energy metabolism and healthy function of the nervous system. A particularly suitable diet should comprise vitamins, minerals, unsaturated fatty acids, amino acids, secondary plant metabolites, including anti-oxidative agents, phytonutrients or essential or semi-essential nutrients.

For a pain-reducing, anti-inflammatory diet particularly vitamins of the group of vitamin, C, vitamin D, vitamin E, vitamin K and B vitamins (thiamine, riboflavin, nicotinamide, pantothenic acid, pyridoxine, biotin, folate, and/or vitamin B₁₂) should applied in sufficient amounts.

Important minerals are selected from group of magnesium, calcium, potassium, sodium, copper, manganese, zinc, selenium, and boric acid / boron.

The vitamins and minerals should be present in the diet in sufficient quantities so that no deficiency symptoms occur. Appropriate recommendations for the daily amount of these nutrients are issued by the German Society for Nutrition (Deutsche Gesellschaft für Ernahrung e.V., Referenzwerte für die Nährstoffzufuhr, 2. Auflage, 7. Aktualisierte Ausgabe, 2021).

A further group of useful nutrients for a pain-reducing, anti-inflammatory diet are unsaturated fatty acids, in particular omega-3 fatty acid; docosahexanoic acid (DHA), eicosapentenoic acid (EPA), alpha-lipoic acid and lecithine.

A preferred diet should be also rich on amino acids selected from the group of L-tyrosine, arginine and glycine. Additional amino acids for example theanine, cystine, taurine, or mixtures thereof are enriched in a preferred diet.

Further compounds that should be present in a pain-reducing, anti-inflammatory diet are N-acetyl-L-cysteine, gamma-amino-butyric acid (GABA), S-adenosylmethionine (SAMe), ubiquintol (coenzyme Q10), NADH, resveratrol, lutein, lycopene, choline, and carnitine.

Phytonutrients, including secondary plant metabolites, which are particularly useful in a pain-reducing, anti-inflammatory diet are for example antioxidants, anthocyanidines, flavonoids, flavones, isoflavones, catechins, anthocyanidins, isothiocyanates, carotenoids, allyl sulfides, polyphenols, resveratrol, lutein, or lycopene. Particularly preferred secondary plant metabolites are oligomeric procyanidins and oligomeric proanthocyanidins (OPCs).

For a preferred diet also plants, particularly herbs, spices, fruits, vegetables and legumes, or extracts, oils, powders, or compounds thereof can be used, for example from the group boswellia serrata, curcuma longa, grape seeds (in particular with OPCs), devil's claw, or cat's claw. Further tomatoes, olive oil, green leafy vegetables, such as spinach, kale, and collards, nuts like almonds and walnuts, or fruits such as strawberries, blueberries, cherries, and oranges are regarded as anti-inflammatory foods. Fatty fish like salmon, mackerel, tuna, and sardines have also anti-inflammatory effects.

The recommended nutrients for a pain-reducing, anti-inflammatory diet can be supplied by selecting suitable foods or by addition of the respective components via nutrition supplements.

### Description of the figures:

Figure 1: Changes in tissue creatine levels in patients suffering under Post-COVID. Left columns representing the group of patients receiving creatine, right columns representing the placebo group.

### Working examples:

A study was conducted to evaluate effectiveness and safety of replenishing creatine via dietary supplementation in patients suffering under Post-COVID after a SARS-CoV-2 infection. This study employed a parallel-group, randomized placebo-controlled double-blind design. The allocation ratio to experimental group (creatine) and control group (placebo) was set at 1:1. The eligibility criteria for patients to be included in the trial were: age 18-65 years, COVID-19 positive test within last 3 months (as documented by valid PCR or antigen test), moderate-to-severe fatigue and at least one of additional Post-COVID-19 related symptoms such as anosmia, ageusia, headache, and concentration difficulties. Exclusion criteria were other pulmonary and cardiovascular conditions, and history of dietary supplement use during the 4 weeks before the trial commences.

The study was conducted in compliance with the Declaration of Helsinki (7th revision). The data presented until now were collected at FSPE Applied Bioenergetics Lab at the University of Novi Sad, from October 2021 to January 2022. The experimental (creatine) group received 4 grams of creatine monohydrate per day, while control group (placebo) received equivalent amount of inulin. The participants were asked to take the intervention one time per day during breakfast, with experimental or control powder stirred in 250 mL of lukewarm water and consumed immediately afterwards. Both interventions were similar in appearance, texture, and sensory characteristics. Creatine monohydrate was provided by Alzchem Trostberg GmbH (Trostberg, Germany). The duration of intervention was 6 months, and participants were asked to refrain from using any other dietary supplements during the trial. All outcome measures were determined at baseline (pre-administration) and after 3 and 6 months. The primary outcome was the change in creatine levels in brain at baseline and at 3-month and 6-month follow-up, respectively. The minimal sample size (n = 12) was calculated using power analysis (G*Power 3.1.9.3, Heinrich-Heine-Universitat Düsseldorf), with the effects size set at 0.50 (medium effect), alpha error probability 0.05, power 0.80 for two groups, and two measurements of study outcomes.

The number of participants who were randomly assigned, received intended treatment, and were analyzed for the primary outcome at this time was twelve in total, six in experimental group and six in control group. The participants recruited reported no major side effects of either intervention so far.

Accumulation of creatine in skeleton muscles in Post-COVID patients.

Tissue levels of creatine were measured with proton magnetic resonance spectroscopy (1.5 T Avanto scanner, Siemens, Erlangen, Germany) using matrix head coil in circularly polarized mode, with metabolite spectra in the specific regions of skeletal muscle and brain (vastus medials muscle, thalamus, frontal, precentral, paracentral, and parietal white and grey matter) processed as previously described (Appl Physiol Nutr Metab. 2016 Sep, 41(9):1005-7.).

Changes in tissue creatine levels after 3 months in patients suffering under Post-COVID are summarized in Table 1. The Table shows changes in tissue creatine levels in white matter (brain), thalamus, vastus medialis and grey matter (brain) after intervention with 4 gram creatine per day for 3 months and 6 months compared to creatine level of the placebo group.

**Table. 1. Changes in tissue creatine level (mM)**

| | Creatine | | | Placebo | | |
|---|---|---|---|---|---|---|
| Total creatine (mM) | Baseline | 3 months | 6 months | Baseline | 3 months | 6 months |
| Vastus medialis muscle | 28.2 ±3.0 | 30.0 ±4.2 | 30.6 ±3.4 | 20.6 ±1.9 | 21.1 ±3.1 | 21.9 ±4.4 |
| Thalamus | 5.7 ± 0.5 | 6.3 ± 0.9 | 6.3 ±1.0 | 5.9 ± 1.5 | 6.1 ± 0.8 | 6.0 ± 1.0 |
| Left frontal white matter | 5.9 ± 0.6 | 6.2 ± 0.6 | 6.2 ± 0.5 | 5.8 ± 0.6 | 5.7 ± 0.6 | 5.7 ± 0.4 |
| Right frontal white matter | 5.6 ± 0.8 | 6.6 ± 0.8 | 6.8 ± 1.0 | 5.7 ± 0.8 | 5.6 ± 0.7 | 5.6 ± 0.4 |
| Left frontal grey matter | 6.6 ± 0.4 | 6.9 ± 0.7 | 6.8 ± 0.7 | 6.2 ± 0.7 | 6.2 ± 1.1 | 6.2 ± 0.8 |
| Right frontal grey matter | 6.5 ± 0.7 | 6.2 ± 0.3 | 6.2 ± 0.5 | 6.4 ± 0.7 | 6.3 ± 0.7 | 6.3 ± 0.6 |
| Left precentral white matter | 6.0 ± 0.6 | 6.2 ± 0.8 | 6.1 ± 0.7 | 5.8 ± 0.4 | 5.8 ± 0.3 | 5.9 ± 0.4 |
| Right precentral white matter | 5.8 ± 0.5 | 5.8 ± 0.7 | 6.0 ± 0.7 | 5.8 ± 0.3 | 5.8 ± 0.3 | 5.9 ± 0.2 |
| Left precentral grey matter | 6.7 ± 0.6 | 7.3 ± 0.9 | 7.1 ± 0.7 | 6.5 ± 0.4 | 6.3 ± 0.6 | 6.4 ± 0.7 |
| Right precentral grey matter | 6.8 ± 0.4 | 7.2 ± 0.5 | 7.0 ± 0.7 | 6.7 ± 0.4 | 6.8 ± 0.4 | 6.6 ± 0.4 |
| Left parietal white matter | 5.3 ± 0.6 | 6.0 ± 0.9 | 6.0 ± 0.6 | 5.2 ± 0.4 | 5.1 ± 0.4 | 5.0 ± 0.3 |
| Right parietal white matter | 5.3 ± 0.7 | 6.9 ± 0.9 | 6.8 ± 1.0 | 5.1 ± 0.6 | 5.2 ± 0.8 | 5.3 ± 0.5 |
| Left mesial grey matter | 7.2 ± 0.9 | 8.0 ± 1.2 | 8.0 ± 1.0 | 7.0 ± 0.7 | 7.0 ± 0.8 | 7.0 ± 0.9 |
| Right mesial grey matter | 7.1 ± 0.3 | 8.2 ± 1.3 | 8.1 ± 1.3 | 7.1 ± 0.4 | 7.2 ± 0.5 | 7.1 ± 0.3 |

The differences in percent of the creatine level after three months and the corresponding baseline level in white matter (brain), grey matter (brain), thalamus, and vastus medialis muscle calculated from Table 1 are shown in Figure 1. Left columns representing the group of patients receiving creatine, right columns representing the placebo group. For the values in white matter and grey matter average values are determined from Table 1.

In the randomized controlled trial, it was found that creatine is accumulated in brain and vastus medialis when administered for 3 months. The increased creatine level can be maintained for at least 3 further months by supplemental creatine administration. No changes in tissue creatine values were found in the placebo group.

Effect of creatine as nutrition supplement for Post-COVID patients.

The patient-reported outcomes for COVID-19-related signs and symptoms (e.g. headache, concentrating difficulties, anosmia, ageusia) were evaluated with VAS scale. Fatigue was evaluated with a multidimensional fatigue inventory test (MFI-20 test, Smets E.M. et al., J. Psychosom. Res. 1995, 39(3), 315). The results of the VAS scale survey and of the MFI-20 test are summarized in Table 2. In addition the time to exhaustion of the patients was evaluated by an incremental test until exhaustion on a motorized treadmill. Speed and gradient of the treadmill are increased every third minute, starting at 2.7 km/h (1.7 miles per hour) at a 10% gradient and rising at stage 7 to 9.7 km/h (6 miles per hour) at 22% gradient (Will P.M. and Walter J.D., Am Heart. J., 1999 Dec, 138, 1033). The results of the treadmill evaluation are provided in Table 3.

**Table. 2**

| | Creatine | | | Placebo | | |
|---|---|---|---|---|---|---|
| | Baseline | 3 months | 6 months | Baseline | 3 months | 6 months |
| General fatigue | 12.0 | 11.3 | 12.7 | 12.0 | 11.8 | 12.8 |
| Physical fatigue | 13.5 | 12.7 | 13.2 | 12.5 | 12.7 | 12.8 |
| Mental fatigue | 12.3 | 11.2 | 10.5 | 11.0 | 11.3 | 12.2 |
| Reduced activity | 11.5 | 12.0 | 12.5 | 12.7 | 12.8 | 14.0 |
| Reduced motivation | 13.0 | 13.0 | 12.5 | 12.2 | 13.0 | 13.7 |
| Headache | 3.8 | 0.8 | 0.5 | 4.4 | 2.2 | 0.0 |
| Concentrating difficulties | 4.0 | 1.5 | 0.0 | 2.6 | 0.9 | 0.2 |
| Anosmia | 5.0 | 1.0 | 0.8 | 2.9 | 1.0 | 0.4 |
| Ageusia | 5.0 | 0.2 | 0.0 | 2.0 | 0.0 | 0.0 |

**Table 3:**

| | Creatine | | | Placebo | | |
|---|---|---|---|---|---|---|
| | Baseline | 3-months | 6-months | Baseline | 3-months | 6-months |
| Time to exhaustion (sec) | 894 | 922 | 959 | 879 | 883 | 897 |

The time to exhaustion is significantly increased in patients suffering under post viral fatigue syndrome after a SARS-CoV-2 virus infection. The time to exhaustion of the creatine group is increased 7.3% compared to 2.1% after placebo intake. More important, the creatine group achieves stage 6 in the treadmill test, wherein the placebo group remains at stage 5. The improved endurance is associated with a reduction in fatigue symptoms, particularly with reduced mental fatigue compared to an increase in the placebo group. The reduced motivation worsens over the observation period in the placebo group compared to slight improvement in the creatine cohort. Smaller improvements are achieved by creatine administration compared to the placebo group relating to fatigue symptoms like physical fatigue, reduced activity, and concentration difficulties according to the results presented in Table 2. In addition, creatine induced no major side effects.

## Claims

1. Pharmaceutical composition comprising creatine or physiologically acceptable derivatives and/or salts and or adducts thereof for use in the treatment of post viral fatigue syndrome (PVFS) associated with conditions selected from the group of mental fatigue, reduced motivation, and concentration difficulties.

2. The pharmaceutical composition of claim 1, wherein the PVFS is caused by a coronavirus infection or are symptoms of Post-COVID.

3. The pharmaceutical composition of claim 1 or 2, wherein the composition is used in combination with glucose.

4. The pharmaceutical composition of any one of claims 1 to 3, wherein the physiologically acceptable creatine derivative is selected from the group consisting of creatine, creatine hydrates, creatine esters or amides, including creatine C₁-C₅-alkyl esters, N-C₁-C₅-alkyl amides, creatinol, creatinol-O-phosphate or a mixture thereof.

5. The pharmaceutical composition of any one of claims 1 to 4, wherein the creatine salts and adducts or the salts and adducts of the physiologically acceptable creatine derivatives are selected from the group consisting of the corresponding acetates, citrates, maleates, fumarates, tartrates, malates, pyruvates, ascorbates, succinates, aspartates, lactates, oxalates, formates, benzoates, phosphates, sulfates, chlorides, hydrochlorides, of the corresponding potassium, sodium, calcium, magnesium salts, of the corresponding L-carnitine, acetyl-L-carnitine, taurine, betaine, choline, methionine adducts or a mixture thereof.

6. The pharmaceutical composition of any one of claims 1 to 5, wherein the composition comprises creatine monohydrate.

7. The pharmaceutical composition of any one of claims 1 to 6, wherein the daily dose of creatine in the composition is in the range of 7g to 30g.

8. The pharmaceutical composition of any one of claims 1 to 7, wherein the daily dose of creatine in the composition is an accumulation dose in the range of 10g to 30g for an initial accumulation phase and a maintaining dose in the range of 7g to 15g for a subsequent maintaining phase, wherein the accumulation phase has a duration of up to 3 weeks and the maintaining phase has a duration of 1 month to 12 months.

9. Use of a composition comprising creatine or physiologically acceptable derivatives and/or salts and or adducts thereof as dietary supplement or as a supplement for preparation of a diet supporting the recovery from PVFS associated with conditions selected from group of mental fatigue, reduced motivation, reduced activity, and concentration difficulties.

10. The use of claim 9, wherein the PVFS is caused by a coronavirus infection or are symptoms of Post-COVID.

11. The use of claim 9 or 10, wherein the daily dose of creatine in the composition is in the range of 7g to 30g.

12. The use of any one of claims 9 to 11, wherein the daily dose of creatine in the composition is an accumulation dose in the range of 10g to 30g for an initial accumulation phase and a maintaining dose in the range of 7g to 15g for a subsequent maintaining phase, wherein the accumulation phase has a duration of up to 3 weeks and the maintaining phase has a duration of 1 month to 12 months.

13. The use of any one of claims 9 to 12, wherein the used composition comprises creatine monohydrate.
